# EUROPEAN PATENT SPECIFICATION

(11) **EP 4 119 124 B1**
(45) Date of publication and mention of the grant of the patent: **01.10.2025**
(21) Application number: 21185640.6
(22) Date of filing: 14.07.2021
(51) Int. Cl.: A61K 47/36, A61K 9/00, A61K 9/107, A61K 31/05, A61K 31/352

(54) **MICROEMULSION CONTAINING ORALLY DISINTEGRATING FILM COMPOSITIONS WITH ADJUSTABLE PHYSICAL AND RHEOLOGICAL PROPERTIES**
MIKROEMULSION MIT ORAL ZERFALLENDEN FILMZUSAMMENSETZUNGEN MIT ANPASSBAREN PHYSIKALISCHEN UND RHEOLOGISCHEN EIGENSCHAFTEN
MICROÉMULSION CONTENANT DES COMPOSITIONS DE FILM À DÉSINTÉGRATION ORALE AUX PROPRIÉTÉS PHYSIQUES ET RHÉOLOGIQUES RÉGLABLES

(43) Date of publication of application: 18.01.2023
(73) Proprietor: Vektor Pharma TF GmbH, 88524 Uttenweiler (DE)
(72) Inventor: BEA, Manuel, 88499 Altheim (DE); ANDELFINGER, Marc, 88356 Ostrach (DE); MOHN, Sarah, 72535 Heroldstatt (DE); MAUCHER, Julia, 88348 Bad Saulgau (DE); KNÖDLER, Tamara, 88371 Ebersbach-Musbach (DE); WAGNER, Wolfgang, 89233 Neu-Ulm (DE); BECKERT, Thomas, 88447 Warthausen (DE)
(74) Representative: SONN Patentanwälte GmbH & Co KG

(56) References cited:
- WO-A1-2013/085224
- WO-A1-2021/138564
- GB-A- 2 587 621
- US-A1- 2016 317 462
- XIAO LU ET AL: "A new self-microemulsifying mouth dissolving film to improve the oral bioavailability of poorly water soluble drugs", vol. 39, no. 9, 26 September 2012 (2012-09-26), US, pages 1284 - 1290, XP055876082, ISSN: 0363-9045, Retrieved from the Internet <URL:https://www.tandfonline.com/doi/pdf/10.3109/03639045.2012.723716?needAccess=true> DOI: 10.3109/03639045.2012.723716

## Description

### Field of the Invention

The present invention relates to film delivery systems, especially for oral delivery, which can be formed during manufacture in the form of film strips or sheets and subsequently cut into uniform dosage units, each dosage unit being uniform in content and having distributed an active ingredient dispersed in a microemulsion within the polymeric matrix comprising of a mixture of at least two bioedible water-soluble polymers. The combination of these polymers enables a precise control of the chemical, physical and mechanical properties of the orally disintegrating film and allows the manufacturing of products with the various desired functionalities. The active ingredient is uniformly dispersed in a microemulsion within the polymeric matrix.

### Background of the Invention

The route of administration is well known to affect the absorption, distribution, and metabolism parameters of pharmaceutical active ingredients. Drug delivery via intravenous (i.v.) injection is known to permit relatively rapid onset of therapeutic effects, however, i.v. injection is not very practical outside of a clinical setting. Accordingly, the oral route of drug administration is the most suitable and preferred route due to its ease of administration, non-invasiveness, adaptability and most important patient compliance and acceptability, although it typically has a very slow onset of therapeutic effects, and drug potency can be lost due to action of the digestive system and/or first pass metabolism. The oral administration of medicinal and therapeutic agents that occur by absorption across the buccal or sublingual mucosa is an attractive alternative to standard oral administration by ingestion into the gastrointestinal tract, as it can bypass first pass metabolism in the liver as well as degradation in the digestive tract. By the novel approach of fast dissolving drug delivery systems, for example orally disintegrating films (ODF), a beneficial mucosal administration of drugs can be achieved leading to higher control of the actual dosage of the active ingredient and increased patient compliance especially in pediatrics or geriatrics. ODFs are typically the size of a regular postage stamp and disintegrate on a patient's tongue in a matter of seconds for the rapid release of one or more active ingredients. The formulation of dissolvable films is customarily facilitated through aqueous polymer matrices that span a wide molecular weight (MW) range enabling the rapid dissolution and fast release of the active pharmaceutical agent upon immediate hydration by saliva in the oral cavity and thereby providing flexibility to achieve certain physical properties.

In comparison to other oral dosage forms, orally disintegrating films have several special advantages such as a rapid disintegrating and dissolution in the oral cavity caused by a larger surface area which improves the onset of action, lower the dosing, and enhance the efficacy and safety profile of the medicament. Since the oral or buccal mucosa being highly vascularized, drugs can be absorbed directly and can enter the systemic circulation without undergoing first-pass hepatic metabolism enabling a reduction of the dose of the active ingredient concomitant with a minimization of associated side-effects. This feature of orally disintegrating films can be exploited in preparing products with improved oral bioavailability of active ingredients that undergo strong first pass effects.

Furthermore, the administered dose of an orally disintegrating film is precisely controlled through the size of the film strip, because the active ingredient is uniformly distributed in the polymer matrix of the film. Moreover, orally disintegrating films are easy in handling transportation and storage and are not as fragile as e.g. orally disintegrating tablets. Besides, the orally disintegrating film dosage form is preferable for patients suffering from dysphagia, repeated emesis, motion sickness, and mental disorders as they are unable to swallow large quantity of water. Therefore, important practical applications for administration via orally disintegrating films include emergency care situations where rapid administration of drugs by non-skilled personnel could be life-saving; in unconscious patients who may have overdosed or experienced a seizure; in elderly dementia patients with dysphagia; as a facile and convenient route of administration of medication to young children; and for drug delivery of medication to animals.

As an illustration, there are several patent publications, for example EP 1 463 491 A1 (WO 03/030882 A1), EP 2 371 358 A1, EP 3 160 589 A1 (WO 2015/200233 A1), EP 3 106 151 A1, EP 3 106 152 A1, EP 3 452 025 A1 (WO 2017/180707 A1) and WO 2020/014776 A1, that describe application of ODF technology used for the mucosal drug delivery of various pharmaceutical agents by incorporating them in a bio-edible polymeric matrix. However, the orally disintegrating film compositions enclosed in these patents are mainly based on one single film-forming polymer, e.g. pullulan (*cf.* EP 3 452 025 A1, WO 2020/014776 A1) or cellulose derivatives, such as hydroxypropyl cellulose or hydroxypropylmethyl cellulose (*cf.* EP1 463 491 A1, EP 3 160 589 A1), and the properties of the matrix are modified only by using different plasticizers or additives. However, films made exclusively from one polymeric component have low flexibility and suffer from the limitation that in order to put higher drug levels in the film an increased polymer content is necessary to preserve uniformity of the film.

Further developments in the field show that several properties of a thin-film, such as flexibility, tensile strength and overall structural integrity, can be easily varied by the application of a combination of different water-soluble polymers for the formation of the film-matrix.

For example, WO 2013/085224 A1 discloses a water-soluble film comprising sildenafil, pullulan and hydroxypropyl pea starch.

For example, in patent application EP 1 463 490 A1, to Yang et al., ingestible water-soluble film compositions comprising of a glucan and a water-soluble polymer, preferably modified cellulosic materials, is disclosed. By reducing the content of glucan in said disintegrating films, higher amounts of active components can be incorporated and higher structural integrity was achieved compared to conventional glucan-based films.

Furthermore, patent application EP 1 713 450 A1 (WO 2005/074894 A1), to Singh et al., discloses a rapidly dissolving film having significant drug loading capability while providing sustained and controlled release of an active agent. This film is made up of high molecular weight synthetic polymers (polyvinylpyrrolidone, polyethylene glycol) and contains a rapidly dissolving oligomeric material, such as maltodextrin.

Moreover, patent application EP 2 120 895 A2 (WO 2008/089151 A2) to Myers et al., discloses orally disintegrating films with high amounts (at least 30%) of various pharmaceutical active ingredient and the preparation method of such film strips. In most of the film compositions a combination of polyethylene glycol and polydextrose in various ratios was utilized to facilitate the incorporation of such high dosages with a reasonable uniformity and distribution of active ingredient within the film strip.

Despite the above described improvements in orally disintegrating film technology, there is a strong need for orally disintegrating films containing active ingredients with abuse-proof formulations that are easy to manufacture and avoids a substantial number of additives, while at the same time, the desired properties of the ODF such as disintegration times, dissolution times, smooth appearance appropriate for commercialization, strength for withstanding handling, uniform distribution of the active ingredients can be controlled by the composition of the formulation. Furthermore, the incorporation of active ingredients in these orally disintegrating films with high accuracy of the dosage is highly desirable. This uniform distribution of the active ingredient can be achieved by a homogenous distribution of the active ingredient in a microemulsion within an adaptable polymeric matrix comprising of a mixture of two water-soluble polymers.

### Summary of the Invention

According to the present invention an orally disintegrating film composition is provided, the film comprising an active ingredient and a polymeric matrix, comprising a combination of at least two water-soluble film forming polymers to form a polymeric matrix and wherein the active ingredient is uniformly dispersed within a microemulsion comprising an oil medium, water and at least one surfactant and optionally at least one cosurfactant, wherein the at least two water-soluble film forming polymers are a pregelatinized hydroxypropyl pea starch and pullulan and wherein the ratio of pregelatinized hydroxypropyl pea starch and pullulan is between 4:1 to 2:3.

The active ingredient is uniformly dispersed in a microemulsion system within the polymeric matrix and the composition has a disintegration time of 300 s or less, preferably of 180 s or less, especially of 120 s or less and/or an ultimate tensile strength of 1.8 N/mm² or below, preferably of 0.8 N/mm² or below, especially of 0.6 N/mm² or below.

Pea starches have advantageous properties which make them specifically useable in the combination formulation according to the present invention, especially in their pregelatinized hydroxypropylated form which provides to the pea starches - despite their high amylose content - a higher paste viscosity, paste clarity and improved properties with respect to the extent of syneresis (Ratnayake et al., Starch 54 (2002), 217-234). Such products are also commercially available, e.g. from Roquette under the name LYCOAT with two grades LYCOAT RS 780 (developing lower viscosity) and LYCOAT RS 720 (developing higher viscosity) or as granular hydroxypropyl starch (LYCOAT NG 720), wherein pregelatinized hydroxypropylated pea starches developing higher viscosities (with a glass transition temperature of at least 45°C at a water content of 20 g of water/100g of wet substance and a glass transition temperature of at least 120°C at a water content of 10g of water/100g of wet substance, measured e.g. by differential scanning calorimetry (DSC) measurement of the differential heat flow between a polymer sample and an inert reference at atmospheric pressure and at 25°C) are specifically preferred.

Pullulan (CAS NO.: 9057-02-7; E 1204) is produced from starch by the fungus Aureobasidium pullulans.

The preferred viscosity of Pullulan is in the range from 100 mm²/s - 180 mm²/s, preferably 140 - 160 mm²/s, more preferably 140 mm²/s.

The orally disintegrating film contains a surfactant and optionally a cosurfactant, which are used to homogenously disperse the active ingredient within the polymeric matrix. The surfactant and/or cosurfactant may be selected from the group consisting of polyglycolized glycerides, polyoxyethylene glycerides, polyethylene glycol-fatty acid esters, polyethylene glycol glycerol fatty acid esters, transesterfication products of alcohols, polyglycerized fatty acids, glycerol fatty acid esters, polyglycerol fatty acid esters, propylene glycol fatty acid esters, mono and diglycerides, polyoxyethylene-polyoxypropylene block copolymers, polyethylene glycol sorbitan fatty acid esters, sorbitan fatty acid esters, polysorbates, poloxamers, phospholipids, lecithin, phosphatidylcholines, phosphatidylethanolamines, phosphatidylinositols, phosphatidylserines, phosphatidic acids, and mixtures thereof.

The orally disintegrating film composition contains an oily medium to form the microemulsion selected from the group consisting of borage oil, castor oil, coconut oil, cottonseed oil, soybean oil, safflower oil, sunflower oil, castor oil, corn oil, olive oil, palm oil, peanut oil, peppermint oil, poppy seed oil, canola oil, hydrogenated soybean oil, hydrogenated vegetable oils, glyceryl esters of saturated fatty acids, glyceryl behenate, glyceryl distearate, glyceryl isostearate, glyceryl laurate, glyceryl monooleate, glyceryl, monolinoleate, glyceryl palmitate, glyceryl palmitostearate, glyceryl ricinoleate, glyceryl stearate, polyglyceryl-10-oleate, polyglyceryl-3-oleate, polyglyceryl-4-oleate, polyglyceryl-10-tetralinoleate, behenic acid, caprylyic/capric glycerides, lauric acid, linoleic acid, linolenic acid, myristic acid, palmitic acid, palmitoleic acid, palmitostearic acid, ricinoleic acid, stearic acid, soy fatty acids, oleic acid, a-tocopherol, y-tocopherol, vitamin E, and vitamin A, and mixtures thereof.

According to certain embodiments, the orally disintegrating film may additionally include one or more plasticizers, taste masking agents, a flavor agent, a solubilizer, a thickener, a coloring agent, an effervescent agent, an antioxidant, a pH modifying agent, vitamins, minerals, a dietary supplement, or mixtures thereof.

According to certain embodiments, the flavoring and/or taste masking agent of the orally disintegrating film composition is present in an amount up to 10% by weight of the film and is selected from the group comprising any one of kleptose, cyclodextrin, cyclodextrin derivatives, ginger, anise, cinnamon, peppermint, licorice, fruit juice, citric acid, citrate, sweeteners, sucrose, glucose, fructose, mannitol, saccharin, aspartame, sucralose, stevia plant derivatives, honey, or any combination thereof.

According to another aspect of the invention the orally disintegrating film composition of any one of the preceding claims comprises of an active component which is selected from the group consisting of pharmaceutical agents, cosmetic agents, bioactive agents and combinations thereof

According to certain embodiments these active component of the orally disintegrating film composition is present in amounts of up to about 0.01% to about 50% by weight of the total composition, preferably 1% to about 30%, more preferably 10% to about 20%.

According to certain embodiments, the active ingredient of the orally disintegrating film composition is selected from the group comprising Cannabinoids, tetrahydrocannabinol (THC), tetrahydrocannabinolic acid (THCA), cannabidiol (CBD), cannabigerol (CBG) and cannabinol (CBN), or mixture or combinations thereof.

According to certain embodiments the orally disintegrating film composition according to the present invention is essentially free of a gum resin.

In another aspect of the invention the orally disintegrating film composition according to the present invention consists of a single layer.

In certain embodiments the orally disintegrating film composition according to the present invention has a polymeric backing layer.

According to certain embodiments the orally disintegrating film composition according to the present invention has a thickness of the film in a range from about 0.01 to 0.8 mm, preferably 0.1 - 0.6 mm, more preferably 0.3 - 0.5 mm.

According to certain embodiments the orally disintegrating film composition according to the present invention comprises of the active ingredient and a film forming agent, having a percentage relative standard deviation of the assay of the active ingredient in a batch of said orally disintegrating film composition of less than 2.5%, preferably less than 1%, more preferably less than 0.5%.

According to another aspect of the invention the orally disintegrating film composition according to the present invention comprises of the active ingredient and a film forming agent, wherein disintegration time in phosphate buffered saline solution at pH 6.8 and 25 °C is determined by the mixture of two hydrophilic polymers and is less than about 10 minutes, preferably less than about 3 minutes, more preferably less than about 2 minutes.

In certain embodiments, the mechanical and rheological properties of the orally disintegrating film composition according to the present invention are modified by the variation of the ratio of two hydrophilic polymers within the formulation.

### Brief Description of the Drawings

**Figure 1** is a block diagram showing the manufacturing procedure of the orally disintegrating film according to an embodiment of this invention.
**Figure 2** is a block diagram showing an alternative manufacturing procedure of the orally disintegrating film according to an embodiment of this invention.
**Figure 3** is a schematic representation of the casting apparatus and the casting procedure.
**Figure 4A** is the microscope image of the coating mass of Example 4 before the degassing procedure.
**Figure 4B** is the microscope image of the coating mass of Example 4 after stirring at 150 rpm for 18 h.

### Detailed Description of the Invention

As used herein, the terms "orally disintegrating film", "orally dispersible film", "orally disintegrating film strip", "film strip" and "film" refer to sheets of variable dimensions comprising a polymeric carrier matrix, and having any shape, including rectangular, square, or other desired shape. The films described herein are typically thin films with thickness that can range from about 10 microns to about 800 microns, preferably from about 80 to about 600 microns, more preferably from 100 to 300 microns, but may be any desired thickness and size so long as they can be placed comfortably into the oral cavity of the user. Films are a single layer and are typically inherently flexible to enable rapid dissolution in the mouth, permeation through the oral mucosa and entry into the bloodstream via capillaries.

The methods for analyzing the different parameters referred to in the present specification are well available to the person skilled in the art. Preferred methods for determining these parameters are disclosed in the example section. In case of doubt, the parameters are to be interpreted as to be measured according to the methods disclosed in the example section. For example, disintegration time is preferably measured as in the section "Disintegration" in the example section. The rheological properties, especially the ultimate tensile strength and the elongation at break, are preferably determined by the method disclosed in section "Rheological properties" of the example section (according to the DIN EN ISO 527-1 guideline (Plastics - Determination of tensile properties) and (by supplementation) the USP general chapter <881>). The dissolution time/properties are preferably performed by the method described in Ph. Eur. Chapter 2.9.3).

### Components of the ODF

The present invention is related to a mucosal dissolving film as defined in the claims. The properties of the orally disintegrating film composition can be modified by the molecular ratios of the different polymers within the formulation. Furthermore, plasticizers and or other additives known in the field may be used for the manufacturing process to achieve orally disintegrating films with the desired properties.

### • Active agent according to the present invention

A wide variety of medicaments, bioactive active substances and pharmaceutical compositions may be included in the dosage forms of the present invention. Examples of useful drugs include ace-inhibitors, antianginal drugs, anti-arrhythmias, anti-asthmatics, anticholesterolemics, analgesics, anesthetics, anti-convulsants, anti-depressants, anti-diabetic agents, anti-diarrhea preparations, antidotes, anti-histamines, anti-hypertensive drugs, antiinflammatory agents, anti-lipid agents, anti-manics, anti-nauseants, anti-stroke agents, anti10 thyroid preparations, anti-tumor drugs, anti-viral agents, acne drugs, alkaloids, amino acid preparations, anti-tussives, anti-uricemic drugs, anti-viral drugs, anabolic preparations, systemic and non-systemic anti-infective agents, anti-neoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anticoagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

The pharmaceutically active agents employed in the present invention may include allergens or antigens, such as, but not limited to, plant pollens from grasses, trees, or ragweed; animal danders, which are tiny scales shed from the skin and hair of cats and other furred animals; insects, such as house dust mites, bees, and wasps; and drugs, such as penicillin. Cosmetic active agents may include breath freshening compounds like menthol, other flavors or fragrances, especially those used for oral hygiene, as well as actives used in dental and oral cleansing such as quaternary ammonium bases.

Examples of pharmaceutical active ingredients for use in the present invention, include the use of cannabinoids. The term "cannabinoid" refers to a naturally extracted, biosynthetic or chemically synthetic compounds having the same chemical structure as the key metabolites of the Cannabis sativa or Cannabis indica plants, and that have physiological activity as agonists or antagonist of one or more cannabinoid receptors, such as the CB1 and CB2 receptors. The preferred cannabinoids for use in the oral disintegrating film strips can be selected from one or more of the following compounds: tetrahydrocannabinol (THC), tetrahydrocannabinolic acid (THCA), cannabidiol (CBD), cannabigerol (CBG) and cannabinol (CBN). Any form of cannabinoid may be used, for example, a purified crystalline powder, a non-crystalline powder, an oil suspension or oil solution, or even a crude plant extract. Preferably a high purity powder (for example, greater than 90%, such as 95% or even 99%) for example of CBD is used. It has been found that high purity powders, such as synthetically manufactured, pure cannabinoid powders having greater than 99% purity of a single cannabinoid, are particularly advantageous for the therapeutic applications claimed herein.

### • Dosages

The film may be prepared to have a specific amount of active agent, such as a specific amount of drug. The amount of drug included should be consistent across a number of batches so that the ingestible unit can achieve regulatory compliance. Accordingly, the method of preparing an ingestible unit can include: preparing a composition having a biologically active agent at a defined amount; forming the composition into a discrete sheet to have the biologically active agent at a defined amount; and including the discrete sheet in a package. The method may also include: determining a dose of a biologically active agent to be included in a film.

The amount of active per unit area is determined by the uniform distribution of the film. For example, when the films are cut into individual dosage forms, the amount of the active in the dosage form can be accurately defined. This is achieved because the amount of the active in a given area is substantially identical to the amount of active in an area of the same dimensions in another part of the film. The accuracy in dosage is particularly advantageous when the active is a medicament, i.e. a drug.

Drug loading into sheets may be up to about 65% by weight of the sheet, and often up to about 10% to 20% by dry weight. The amount of drug in each sheet can be calculated before or after shaping the individual films or ingestible unit into the size and shape of the dosage form. The amount of drug lost during processing is usually taken into account in order to design the ingestible units and select the appropriate number of sheets to arrive at the predetermined dose.

The film products of the present invention are capable of accommodating a wide range of amounts of the active ingredient. The films are capable of providing an accurate dosage amount (determined by the size of the film and concentration of the active in the original polymer/water combination) regardless of whether the required dosage is high or extremely low. Therefore, the film products and methods of the present invention are also well suited for high potency, low dosage drugs. This is accomplished through the high degree of uniformity of the films.

### • Film Forming Polymers

The polymeric matrix of the orally disintegrating film composition is formed by a combination at least two bio-edible and water-soluble or water-swellable polymers.

One of the film-forming polymers is used to predetermine the mechanical and physical properties of the orally disintegrating film strip. This film-forming polymer is pregelatinized hydroxypropyl pea starch.

The second film-forming agent, such as a film-forming polymer, is used to vary the chemical and physical properties of the disintegrating film strip, defined through the properties of the first polymer. By this combination of two film-forming polymers, orally disintegrating films with the desired properties can be achieved. The second film-forming polymer is pullulan.

In certain embodiments, combined content of all film-forming agents is used in a range of about 20-80 wt%, for example 40-60 wt%, such as about 50 wt%.

The ratio of the first film-forming polymer to second water-soluble polymer is between 4:1 to 2:3.

### • Oil for Microemulsion

The active pharmaceutical ingredient is uniformly dispersed in a microemulsion within the polymeric matrix to achieve highly accurate dosage of active ingredient within the orally disintegrating film strip. The microemulsion is formed in three steps: first dissolution of the active ingredient in a carrier oil or use of an oil extract of the active ingredient, second addition of the surfactants and/or cosurfactants in water followed by a homogenization procedure. The application of dissolution in an oily medium and formation of a microemulsion is extremely beneficial for poorly water-soluble active ingredients, because the bioavailability of these hydrophobic active agents is strongly increased.

The carrier oil or diluent, may include any edible oil that is Generally Recognized as Safe (GRAS) or pharmaceutical quality. For example, castor oil, hemp oil, olive oil, medium chain triglyceride (MCT) oil or safflower oil may be used. Other edible oils may be suitable, including major food oils such as vegetable, canola, peanut, almond, coconut, avocado, sesame, corn, cottonseed, palm, peppermint, rapeseed, soybean, and sunflower; nut oils such as almond, beech nut, brazil nut, cashew, hazelnut, macadamia, mongongo nut, pecan, pine nut, pistachio, and walnut, citrus oils such as grapefruit seed oil, lemon oil, and orange oil; oils from melon and gourd seeds including from the members of the Cucurbitaceae family, such as bitter gourd oil, bottle gourd oil, buffalo gourd oil, butternut squash seed oil, egusi seed oil (from Cucumeropsis mannii naudin seeds), pumpkin seed oil and watermelon seed oil; food supplement oils such as Agai oil (from fruit of several species of the Agai palm), black seed oil (pressed from Nigel/a sativa seeds), blackcurrant seed oil (from Ribes nigrum seeds), borage seed oil (from Borago officinalis seeds), evening primrose oil (from Oenothera biennis seeds), and flaxseed oil (or linseed oil) (from Linum usitatissimum seed); or other known edible oils such as amaranth seed oil (from sees of grain amaranth species including Amaranthus cruentus and Amaranthus hypochondriacus), apricot oil, apple seed oil, argan oil (from the seeds of the Argania spinosa), artichoke, avocado, babassu oil (from the seeds of the Attalea speciosa), ben oil (from the seeds of Moringa oleifera), Borneo tallow nut oil (extracted from the fruit of species of genus Shorea), cape chestnut oil (also called yangu oil), carob pod oil (Algaroba oil), cocoa butter, cocklebur oil (from species of genus Xanthium), cohune oil (from Attalea cohune), coriander seed, date seed oil, dika oil (from Irvingia gabo mensis seeds), false flax oil (from Camelina sativa seeds), grape seed oil, hemp oil, kapok seed oil (from Cieba pentandra seeds), kenafseed oil (from Hibiscus cannabinus seeds), lallemantia oil (from Lallemantia iberica seeds), mafura oil (from Trichilia emetica seeds), marula oil (from Sclerocarya birrea kernel), meadowfoam seed oil, mustard oil, niger seed oil (including from Guizotia abyssinica), nutmeg butter (extracted by expression form the fruit of cogeners of genus Myristica), nutmeg oil, okra seed oil (from Abelmoschus esculentus seeds), papaya seed oil, papaya oil produced by maceration, perilla seed oil, persim mon seed oil (including from Diospyros virginiana), pequi oil (from Caryocar basiliense seeds), pili nut oil (from Canarium ovatum seeds), pomegranate seed oil (from Punica granatum seeds), poppy seed oil, pracaxi oil (from Pentaclethra macro/oba seeds), prune kernel oil, peach kernel oil, quinoa oil, ramtil oil (from one of several species of genus Guizotia abyssinica seeds), rice bran oil, royle oil (from Prinsepia utilis seeds), Sacha inchi oil, Sapote oil, see oil (from Jessenia bataua seeds), shea butter, taramira oil (from arugula or Eruca sativa seeds), tea seed oil (carnelia oil), thistle oil (from Silybum marianum seeds), Tigernut oil (or nut-sedge oil, from the Cyperus esculentus tuber), tobacco seed oil (from Nicotiana species seeds, if purified), tomato seed oil, and wheat germ oil.

In certain embodiments, the carrier fluid is castor oil and or safflower oil. In certain embodiments, about 0.1 to 30 wt% of oil is utilized, for example 10 to 20 wt% of oil. In certain embodiments between 10:1 to 1:10 weight ratio of active ingredient:oil is used, for example, about a 2:1 weight ratio.

### • Surfactant

For the formation of a stable microemulsion of the active ingredient at least one surfactant and/or cosurfactant may be used. The surfactant may include ionic surfactants including anionic and/or cationic surfactants such as sodium dodecyl sulfate (SDS), sodium lauryl sulfate (SLS), benzalkonium chloride, benzthonium chloride, benzyldimethyldodecyl ammonium bromide (BDDAB), and non-ionic surfactants such as polysorbate 80, sorbitan monooleate, lecithins, glycolipids, fatty alcohols, fatty acids, esters of fatty alcohols and fatty acids, sorbitan esters, polyols such as polysorbates, sorbitans, long-chain aliphatic acids that can be saturated or unsaturated and having more than 6 carbons, such as stearic acid, lauric acid, oleic acid, lineoleic acid, PEG-40 hydrogenated castor oil, sodium deoxycholate, poloxamers, bile salts such as sodium taurocholate, phospholipids, phosphatidylcholines, phosphatidylethanolamines, phosphatidylinositols, phosphatidylserines, phosphatidic acids, polyglycolized glycerides, polyoxyethylene glycerides, polyethylene glycol-fatty acid esters, polyethylene glycol glycerol fatty acid esters, transesterfication products of oils and alcohols, polyglycerized fatty acids, glycerol fatty acid esters, polyglycerol fatty acid esters, propylene glycol fatty acid esters, mono and diglycerides, polyoxyethylene-polyoxypropylene block copolymers, polyethylene glycol sorbitan fatty acid esters, sorbitan fatty acid esters derivatives thereof, and combinations thereof. Other suitable surfactants could also be used, and preferably, low molecular weight surfactants are advantageous.

The amounts used of such surfactants can range from about 0.5 wt% to about 20 wt%, and more preferably between 1 wt% to about 10 wt% of total, for example, 4 wt% to 8 wt% of total, although it is possible to use surfactants that are out of this range.

### • Plasticizers

A plasticizer may be used in the formulation of the oral disintegrating film strip. The plasticizer should be a food-grade or pharmaceutical-grade compound, for example one or more of the include low molecular weight polyols such as glycerol, propylene glycol, polyethylene glycols, monosaccharides such as xylitol, erythritol, mannitol, sorbitol; disaccharides such as sucrose, lactose, and maltose; oligosaccharides such as glycogen, inulin, and dextrins such as maltodextrin and similar compounds; citrate derivatives such as citric acid, citrate, tributyl citrate, triethyl citrate, acetyl citrate, citrate ester, triacetin, castor oil, medium-chain triglycerides (MCT) of fatty acids (also known as MCT oil), and various plant oils of low viscosity such as soybean oil, canola oil, corn oil and similar oils, mineral oil, myglyol, derivatives thereof, and combinations thereof. The amounts used of such plasticizer can range from about 0.5 wt% to about 25 wt%, and more preferably between 5 wt% to about 20 wt% of total, for example, 10 wt% to 15 wt% of total, although it is possible to use plasticizers that are out of this.

### • Coloring/Flavoring/ Sweetening/ Taste masking/ Stabilizing and Thickening Agents

Coloring agents may also be present in the oral disintegrating film and could include titanium dioxide, and dyes suitable for food such as those known as F.D. & C. dyes and natural coloring agents such as grape skin extract, beet red powder, beta-carotene, annato, carmine, turmeric, paprika, etc. The colorings agent, if present in the film, may range from about 0 to about 2.5 weight percent of the total composition, for example, 1 wt% of the total composition. Flavoring agents may also be present in the oral disintegrating film and could include synthetic flavor oils and flavoring aromatics and/or natural oils, extracts from plants, leaves, flowers, fruits and so forth and combinations thereof. These may include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil. Also useful as flavors are vanilla, citrus oil, including lemon, orange, grape, lime and grapefruit, and fruit essences, including apple, pear, peach, strawberry, raspberry, cherry, plum, pineapple, apricot and so forth. Flavors which have been found to be particularly useful include commercially available orange, grape, cherry and bubble gum flavors and mixtures thereof. Flavor acids, such as citric acid, malic acid, tartaric acid, lactic acid, and ascorbic acid, may also be used to provide acidic tastes. Flavors may be present in an amount ranging from about 0.5% to about 10.0% by weight based upon the weight of the composition.

Sweetening or taste masking agents may also be present in the oral disintegrating film and could include natural sweeteners, for example, sucrose, stevia, corn syrup, honey, maple syrup, erythritol, maltitol, mannitol, dextrose, fructose, glucose, xylitol, sorbitol, isomalt, and the like, or combinations thereof; and artificial sweeteners, for example, aspartame, sucralose, acesulfame potassium, saccharin, saccharin cyclamate, kleptose, cyclodextrin, cyclodextrin derivatives, and the like, or combinations thereof. The amount of the one or more sweetener agents may range from 0 to 2.5 weight percent, for example, about 1 wt% based on the total weight of the composition.

The oral disintegrating film strip may also include one or more of a permeation or permeability enhancer, a bitter blocker, a filler, an effervescent agent, an anti-oxidant, a disintegrating agent, a pH modifying agent, a buffer, a complexing agent, a bioadhesive, a sheet adhesive, an emulsifying agent, a crystallization inhibitor, a preservative, a unique identifying agent such as a UV active fluorophore, and an antimicrobial.

An anti-oxidant may also be added to the film to prevent the degradation of an active, especially where the active is oxygen- or photosensitive.

### Preparation method of the ODFs

### • Description of formation of micro emulsion, homogenization, casting, drying

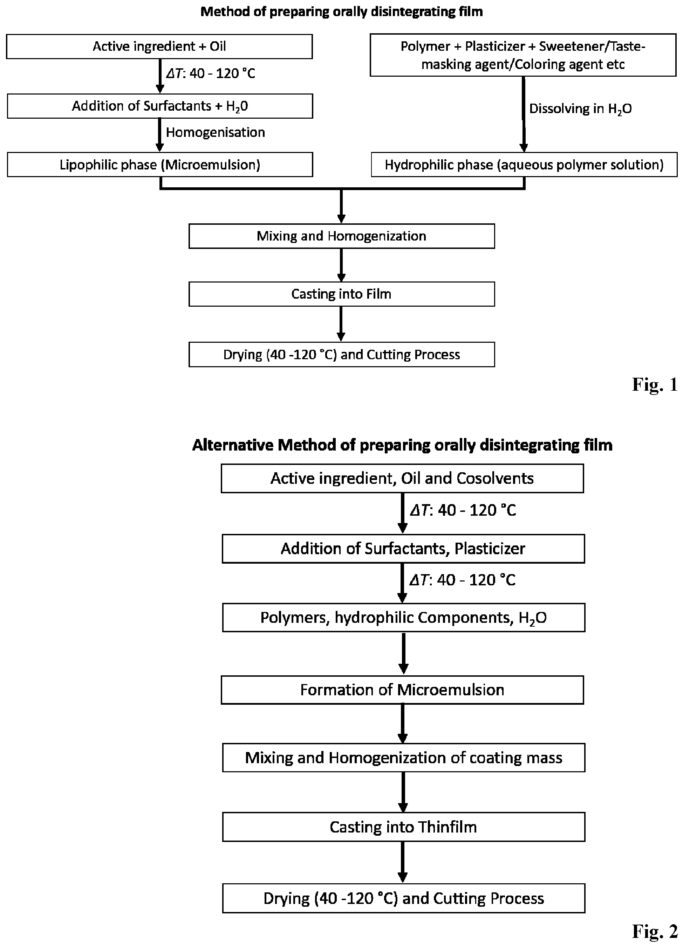

The general procedure for the manufacturing of the orally disintegrating film composition is schematically illustrated in Figure 1.

The used active ingredients (powder form or already dissolved in a hydrophilic solvent or oily medium) are homogenously suspended in a lipophilic medium, e.g. an oil or lipophilic solvent, by stirring at a defined temperature (20-80 °C). After that, the surfactants and/or the cosurfactants and a distinct amount of deionized water are added to the reaction vessel and the mixture is stirred again until all components are homogenously dispersed and the pre-emulsion is formed. This pre-emulsion can be formed instead in a one-step procedure by placing all the above described components in a reaction vessel and homogenization of the mixture by stirring at a defined temperature (20-80°C). In both cases, the homogenization of this pre-emulsion can also be achieved by applying mechanical agitation at high shear rates for example by using an Ultra-Turrax^{®} or by ultrasonication. In another reaction vessel at least two different hydrophilic polymers and if applicable additional surfactants, plasticizers and the other excipients, i.e., coloring, flavoring, sweetening, taste-masking, thickening and stabilizing agents, are dissolved or dispersed in water. Subsequently, the previously described pre-emulsion is added to this mixture and the dispersion is homogenized by stirring, by using an Ultra-Turrax^{®} or by applying ultrasonication. By this homogenization procedure, the uniform distribution of the active ingredient in a microemulsion within the coating mass is achieved.

As an alternative preparation method of the coating mass a one-pot process can be applied for the mixing of the components (schematically illustrated in **Figure 2****).** In this manufacturing process the microemulsion is formed during the last step after addition of the polymers. This alternative one-pot process is described in the following: In this alternative manufacturing process, the active ingredient is solved in the lipophilic and/or oily medium at a minimum of 30 °C. After that, the lipophilic or in oil suspendable components (e.g. the emulsifiers, (co)-surfactants, plasticizers etc.) are added and the mixture is stirred at a minimum of 30 °C until all components are homogenously dispersed. Subsequently, the mixture of the hydrophilic polymers and the other hydrophilic components are added together with a sufficient amount of water. The resulting reaction mixture is homogenized for a minimum of 15 minutes either by stirring with high rotation speeds (400 - 1500 rpm) or by mechanical agitation at high shear rates for example by using an Ultra-Turrax^{®} or by applying ultrasonication to the dispersion. The mixing step of the coating mass is conducted until the lipophilic phase is uniformly distributed and a stable and homogenous microemulsion is formed.

After the mixing step, the further procedure is for both above described manufacturing processes analogous, as follows: The resulting dispersion, wherein the active ingredient is distributed in a microemulsion system, is optionally degassed by stirring at room temperature or at 30 - 50 °C for 1-24 h or by stirring briefly under reduced pressure. After that, a sufficient amount of water is added to exactly adjust the desired solid content (20-80 wt% w/w) and viscosity (100-10000 cP) of the final coating mass.

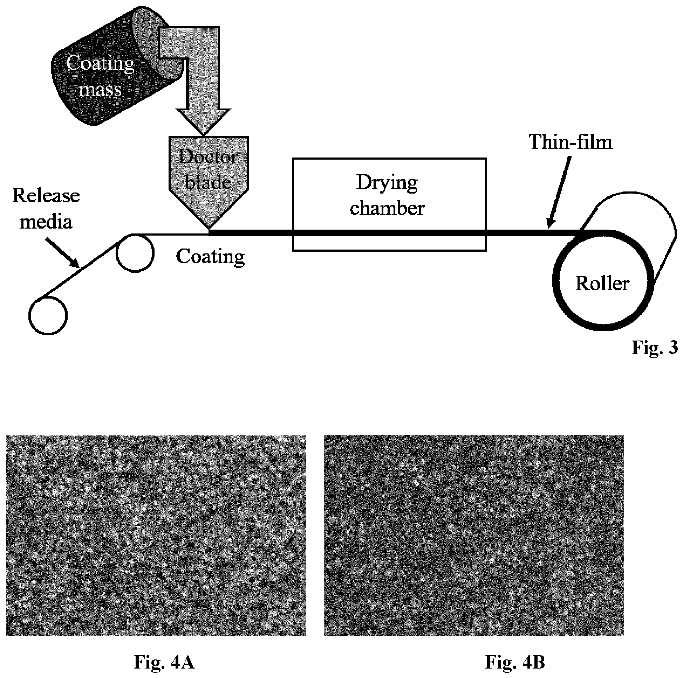

The stable dispersion was then preferably cast to form an oral disintegrating film. The oral disintegrating film was formed by placing the stable dispersion onto a clean receiving substrate or the like to form the oral disintegrating film. The stable dispersion may be dispensed from a suitable apparatus equipped with a doctor blade as would be known in the art, such as a dispensing apparatus, onto the receiving substrate. The stable dispersion may be poured, injected, or otherwise deposited onto the receiving surface by any suitable process or means. The receiving surface can be of any suitable type, such as a tray on a conveyor belt, or a conveyor belt itself. Otherwise, the receiving substrate/surface can be provided in the process e.g. as a continuously moving surface in the form of a release liner which can be easily removed in the course of the further process. The release liner itself can also be constantly moved by a drive roller to guarantee its continuous motion during the casting process **(****Figure 3****).**

The cast stable dispersion was dried by being subjected to preferably an infra-red heater system or alternatively heat or hot air in order to form the oral disintegrating films. The apparatus can move the receiving surface through e.g., a convention style oven, or alternatively, the drying can be done in a batch process. In other embodiments, the drying was done with a substrate temperature set to 40-110 °C, for example to about 80 °C, with an infrared-heater system. In a typical embodiment, drying was performed for between about 1 and 15 minutes. After the oral disintegrating film has been dried, it would have a uniform thickness in the range of 100 to 800 microns. The film can be cut into any desirable shape and size, e.g. by a knife/scalpel or by a LASER cutting process. Alternatively, the stable dispersion may be cast into forms of the desired size and shape, which eliminates the cutting step.

One skilled in the art will appreciate that, for this and other processes and methods disclosed herein, the functions performed in the processes and methods may be implemented in differing order. Furthermore, the outlined steps and operations are only provided as examples, and some of the steps and operations may be optional, combined into fewer steps and operations, or expanded into additional steps and operations without detracting from the essence of the disclosed embodiments.

In one embodiment, the oral disintegrating film made with the method of the present invention and incorporating Cannabidiol (CBD) as the active ingredient, was found to have percent relative standard deviation of the CBD concentration per strip of less than 2.5%, preferably less than 1.0% and more preferably less than 0.5%. This is as compared to prior art methods, where, in the absence of the creation of a stable microemulsion during the manufacturing process, distribution of the active ingredient can be much less uniform in the final product.

In certain embodiments, it is advantageous to have a single layer film strip, as described above. A single layer strip typically has a quicker absorption profile. However, in certain other embodiments, a multi-layer film strip is advantageous. A multi-layer film strip may be advantageous if it is desired to keep one pharmaceutically active ingredient away from a second pharmaceutically active ingredient, for example, where the ingredients may interact or degrade. In such an example, a three-layer laminate may be utilized, with an inert layer sandwiched between the two active layers. In other embodiments, it may be advantageous to manufacture a multi-layer film strip having a single active pharmaceutical layer, manufactured as described above, overtop of a mucoadhesive layer, which aids in the adhering of the strip to the oral lingual, sublingual or buccal mucosa of the patient. A multi-layer film strip may also comprise an abrasive layer for enhancing mucosal, sublingual, or buccal delivery, or permeation enhancers, particularly for animal health applications.

According to a specifically preferred aspect, the present invention relates to an orally disintegrating film composition comprising a Cannabinoid (e.g. Cannabidiol) as active ingredient and a polymeric matrix, which is comprising of a combination of at least two water-soluble film forming polymers to form a polymeric matrix and wherein the active ingredient is uniformly dispersed in a microemulsion within the polymeric matrix and wherein the at least two water-soluble film forming polymers are a pregelatinized hydroxypropyl pea starch and pullulan.

According to a preferred embodiment, the orally disintegrating film composition has a disintegration time of 240 s or less, preferably of 90 s or less, especially of 60 s or less.

According to a preferred embodiment, the orally disintegrating film composition has an ultimate tensile strength of 0.5 N/mm² or below, preferably of 0.4 N/mm² or below, especially of 0 3 N/mm² or below

According to another aspect, the present invention also relates to a method for producing an orally disintegrating film composition according to the present invention, wherein a preparation comprising a Cannabinoid (e.g. Cannabidiol) is mixed with a preparation of the first film-forming polymer, which is pregelatinized hydroxypropyl pea starch, and a preparation of the second film-forming polymer, which is pullulan, by stirring with at least 100 rpm for at least 10 min at a temperature of at least 40°C.

Preferably, stirring is performed with at least 500 rpm for at least 20 min at a temperature of 40°C to 65°C, preferably of 45°C to 55°C.

Preferably, the mixture is degassed after mixing for at least 1 h, preferably for at least 2 h.

According to a preferred embodiment, the degassed mixture is adjusted to the desired solid content by adding an aqueous medium, preferably demineralized water.

Preferably, the mixture is casted and dried at a temperature of at least 60°C, preferably at a temperature of 65°C to 100°C.

According to a preferred embodiment, the mixture is casted and dried to a film with a thickness of 10 to 800 µm, preferably of 200 to 700 µm, especially of 300 to 400 µm.

### Uses of thin films

### • Incorporation of API, mucosal application

The oral disintegrating film strips can be used to treat and/or prevent a disease, a disorder, or a condition. The active ingredient in the oral disintegrating film strip will dictate the disease, disorder, or condition. The disintegration of the film can occur within about 60 seconds to minutes upon coming in contact with water, such as the saliva, stomach fluid, or delayed to occur in the small or large intestines. The disintegration may also occur immediately upon coming in contact with saliva, producing a predefined breakage pattern into smaller pieces for easier mucosal delivery. If the orally disintegrating film contains a cannabinoid, it can be used for treatments known in the art. Oral disintegrating film strips comprising a cannabinoid, for example CBD and/or THC, may be used to treat and/or prevent appetite suppression, neuropathic pain, nausea, intraocular pressure, anxiety, sleep disorders such as insomnia, seizures, and any number of other diseases, disorders, or conditions.

Specifically, CBD could be used for treating symptoms of rheumatoid arthritis and other autoimmune diseases, diabetes, nausea, bowel disorders, inflammation, provide neuroprotective effects, and provide anti-cancer effects, and reduce many other hard-to-control side effects of other medications

THC can be used to treat aches, pains, backache, muscle stiffness, joint pain, inflammation, and reduce many other hard-to-control side effects of other medications or anticancer treatments. The THC can be used to provide anti-emetic, appetite enhancing, relaxation, analgesia, and therapeutic use in Tourette's syndrome, treat dystonia and tardive dyskinesia, treat nausea, treat vomiting, treat anorexia, treat cachexia, treat spasticity, treat movement disorders, treat Multiple Sclerosis, treat pain (rheumatoid arthritis, cancer pain, headache, menstrual pain, chronic bowel inflammation and neuralgias), treat glaucoma, treat epilepsy, treat asthma, treat addiction, treat substance dependency, treat withdrawal symptoms, treat psychiatric symptoms, treat autoimmune diseases, treat inflammation (e.g. ulcerative colitis, arthritis), and many others, and may be used to treat pruritus, hiccup, attention deficit syndrome, high blood pressure, tinnitus, chronic fatigue syndrome, restless leg syndrome, insomnia, depression, anxiety, stress, situational sadness (e.g., from death in family), and any others.

### • Animal health application

Potentially, the orally disintegrating films can be used for an animal health application. When creating film strips intended for animal consumption, different flavoring agents may be used to improve the desirability and palatability of the oral disintegrating film. Preferred flavoring agents are those that are not derived from animal sources. In various embodiments, flavoring components derived from fruit, meat (including, but not limited to pork, beef, chicken, fish, poultry, and the like), vegetable, cheese, bacon, cheese-bacon and/or artificial flavorings may be used. A flavoring component is typically chosen based upon consideration related to the animal that will be ingesting the film strip. For example, a horse may prefer an apple flavoring component, while a dog may prefer a meat flavoring component. Although flavoring components derived from non-animal sources are preferred, in some embodiments, natural flavors containing beef or liver extracts, etc., may be used such as braised beef flavor, artificial powdered beef flavor, roast beef flavor and corned beef flavor among others. Non-animal flavoring agents include, but are not limited to, artificial beef flavors, flavors derived from plant proteins such as soy protein to which artificial flavoring has been added (e.g. soy-derived bacon flavoring), and flavors derived from plant proteins such as soy protein with no artificial flavoring.

When the disintegrating film strips are generated for animal health use, the active ingredient can include any known veterinary pharmaceutical or therapeutic agent or combination. The veterinary disintegrating thin strips may or may not include cannabinoids.

### Examples

Specific ranges, values, and embodiments provided in the examples below are for illustration purposes only and do not otherwise limit the scope of the invention, as defined by the claims. The invention will now be explained more specifically with reference to the following examples, which are given for illustration of the invention and are not intended to be limiting thereof.

### General Procedure for making orally disintegrating thin films

The general procedure for the manufacturing of the orally disintegrating film composition is schematically illustrated in **Figure 1****.** The used active ingredient (powder form or already dissolved in lipophilic solvent or oil) is added to the oily medium in a reaction vessel equipped with overhead stirrer. The mixture is heated at a minimum of 30 °C until the active ingredient is homogenously suspended in the oily medium. The surfactants, e.g. Lipoid P LPC 80 and SPAN 80, are added to the reaction vessel and the mixture is stirred at a minimum of 30 °C until all components are homogeneously dispersed. After that, sufficient deionized water is added to the mixture and the resulting reaction mixture is homogenized for a minimum of 15 minutes either by stirring with high rotation speeds (400-1500 rpm) or by mechanical agitation at high shear rates for example by using an Ultra-Turrax^{®} or by applying ultrasonication to the dispersion. In another reaction vessel equipped with an overhead stirrer the mixture of hydrophilic polymers, e.g. modified starch and pullulan in the respective ratios, and the other excipient, e.g. glycerol and citric acid, are suspended in water with a stirring rate of 300-1000 rpm. The previously described lipophilic phase containing the microemulsion of the active ingredient is added to this suspension and the mixture is stirred with 300-1000 rpm for 15 to 60 min.

As an alternative preparation method of the coating mass a one-pot process can be applied for the mixing of the components (schematically illustrated in **Figure 2****).** In this manufacturing process the microemulsion is formed during the last step after addition of the polymers. This alternative one-pot process is described in the following: In this alternative manufacturing process, the active ingredient is solved in the oily medium at a minimum of 30 °C. After that, the lipophilic or in oil suspendable components (e.g. the emulsifiers, surfactants, plasticizers etc.) are added and the mixture is stirred at a minimum of 30 °C until all components are homogenously dispersed. Subsequently, the mixture of the hydrophilic polymers and the other hydrophilic components are added together with a sufficient amount of water. The resulting reaction mixture is homogenized for a minimum of 15 minutes either by stirring with high rotation speeds (400 - 1500 rpm) or by mechanical agitation at high shear rates for example by using an Ultra-Turrax^{®} or by applying ultrasonication to the dispersion. The mixing step of the coating mass is conducted until the lipophilic phase is uniformly distributed and a stable and homogenous microemulsion is formed.

After this mixing step, the further procedure is for both above described manufacturing processes very similar, as follows: The resulting dispersion is optionally degassed by stirring at room temperature or at 30 - 50 °C for 1-24 h or by stirring briefly under reduced pressure. After that, a sufficient amount of water is added to exactly adjust the desired solid content (20-80 wt% w/w) and viscosity (100-10000 cP) of the final coating mass. Using a mechanical casting apparatus equipped with doctor blade assembly, the stable coating mass is cast onto a PET substrate at the rate of 0.05-1.0 m/min, followed by drying at 50-90 °C using an IR-drying apparatus or a conventional heating system **(****Figure 3****).** With this drying process sufficient amounts of water in the composition are removed to produce a uniform thin film having an approximate thickness in the range of 20 to 500 microns. The film was subsequently cut into film strips of the desired size and shape either by carefully cutting the laminates with a knife/scalpel or by using a punching machine or by using a LASER cutting process.

Using the above described ingredients in variable ranges, the manufacturing of an oral disintegrating film using the above described general method involves the formation of a stable wet dispersion, which after casting onto a substrate, produces a thin, semi-opaque film strip with improved mechanical and chemical properties depending on the mixing ratio of the polymers. Thus, the disintegration time or the tensile strength of the orally disintegrating film can be adjusted to produce films with improved properties compared to prior art. For example, for the composition described in the examples below the disintegration time can be varied in a range from 1.5 min to 13 min and the ultimate tensile strength from 0.96 to 1.55 N/mm². If an active ingredient, e.g. a Cannabinoid, is included in the orally disintegrating film (see also Example 4 and Example 5) this general manufacturing procedure ensures a very uniform distribution of active ingredient across the film surface, because the active ingredient is uniformly distributed in a microemulsion within the polymeric matrix. Thus, the thin film will have a low relative standard deviation (RSD) of the concentration of active ingredient, resulting in more accurate dosing and thereby resulting in more consistent therapeutic efficacy, as compared to film strips of the prior art, which do not have methods that use the formation of a microemulsion.

### Example 1: Compositions and method of making oral disintegrating films with a ratio of starch:pullulan of 80:20 without active ingredient

In a 500 mL reaction vessel equipped with an overhead stirrer, 10.0 g Lipoid P LPC 80 and 8.01 g SPAN 80 were suspended in 8.00 g castor oil at 45 °C. Subsequently, 36.0 g deionized water was added and the mixture was stirred with 700 rpm at 45 °C for 15 min. In another reaction vessel equipped with an overhead stirrer 52.2 g Lycoat NG 720, 13.1 g pullulan (mass ratio of 80:20), 11.1 g glycerol and 2.22 g citric acid were suspended in 120.0 g water with a stirring rate of 700 rpm for 20 min. 51.7 g of the previously described lipophilic phase containing castor oil, Lipoid P LPC 80, SPAN 80 and water were added to the suspension and the resulting mixture was stirred at room temperature with 500 rpm for 30 min. The resulting dispersion was degassed by stirring at 150 rpm at room temperature for 18 h. After that, 61.1 g water were added to achieve the desired solid content (ca. 35 wt%) of the final coating mass. Using a mechanical casting apparatus equipped with doctor blade assembly and convection heating chamber, the stable dispersion is cast onto a PET substrate at the rate of 0.1 m/min and a gap width of 450 µm, followed by drying at 70-90 °C using an IR-drying apparatus. With this drying a uniform thin film having a thickness of about 271 µm and a dry coating weight of 30.5 mg/cm²was produced.

**Table 1. Compositions of the orally disintegrating thin film with a ratio for lycoat:pullulan of 80:20.**

| Example 1 | |
|---|---|
| Component | wt% in dry mass |
| Castor oil | 6.67 |
| Lipoid P LPC 80 | 8.34 |
| SPAN 80 | 6.67 |
| Lycoat NG 720 | 52.0 |
| Pullulan | 13.0 |
| Glycerol | 11.1 |
| Citric acid | 2.22 |
| Total | 100 |

### Example 2 and 3: Oral disintegrating films with different ratios of starch and pullulan without active ingredient

Examples 2 and 3 are compositions of orally disintegrating films prepared by using the same method as in example 1, with different ratios of the film-forming polymers Lycoat and pullulan to modify the physical and chemical properties of the films. The following ratios of Lycoat : pullulan were used without changing any quantity or component of the composition: 60:40 (Example 2), 40:60 (Example 3). The detailed formulations of the examples 2 and 3 are summarized in Table 2.

**Table 2. Compositions of the orally disintegrating thin films with modified ratios of the film-forming polymers starch:pullulan (Example 2: 60:40, Example 3: 40:60).**

| Example 2 | | Example 3 | |
|---|---|---|---|
| Component | wt% in dry mass | Component | wt% in dry mass |
| Castor oil | 6.67 | Castor oil | 6.67 |
| Lipoid P LPC 80 | 8.34 | Lipoid P LPC 80 | 8.34 |
| SPAN 80 | 6.67 | SPAN 80 | 6.67 |
| Lycoat NG 720 | 39.0 | Lycoat NG 720 | 26.0 |
| Pullulan | 26.0 | Pullulan | 39.0 |
| Glycerol | 11.1 | Glycerol | 11.1 |
| Citric acid | 2.22 | Citric acid | 2.22 |
| Total | 100 | Total | 100 |

### Example 4: Compositions and method of making an ODF with CBD

In Example 4 Cannabidiol (CBD) is incorporated as active pharmaceutical ingredient in the orally disintegrating film. This example demonstrates that an active ingredient can easily be incorporated and uniformly distributed in the orally disintegrating film composition of the invention. The complete composition of Example 4 is summarized in Table 3. The orally disintegrating film of Example 4 was prepared according to the general procedure described above.

In a 500 mL reaction vessel equipped with an overhead stirrer, 15.0 g Cannabidiol (CBD), 11.30 g Lipoid P LPC 80 and 9.02 g SPAN 80 were suspended in 9.01 g castor oil at 40 °C. Subsequently, 48.7 g deionized water was added and the mixture was stirred with 1000 rpm at 40 °C for 30 min. In another reaction vessel equipped with an overhead stirrer 58.5 g Lycoat NG 720, 29.3 g pullulan, 15.4 g glycerol and 3.07 g citric acid were suspended in 184.5 g water with a stirring rate of 900 rpm for 10 min. 84.9 g of the previously described lipophilic phase containing CBD, castor oil, Lipoid P LPC 80, SPAN 80 and water were added to the suspension and the resulting mixture was stirred at room temperature with 1000 rpm for 15 min. The microscope image of the coating mass **(****Figure 4A****)** shows a very homogenous microemulsion illustrating the uniform distribution of CBD within the coating mass.

After that, 52.8 g water were added to achieve the desired solid content (ca. 35 wt%) of the final coating mass. The resulting dispersion was degassed by stirring at 150 rpm at room temperature for 18 h to remove air bubbles in the coating mass (see microscope image: **Figure 4B****).** Using a mechanical casting apparatus equipped with doctor blade assembly and convection heating chamber, the stable dispersion is cast onto a PET substrate at the rate of 0.1 m/min and a gap width of 450 µm, followed by drying at 70-90 °C and 45% RH using an IR-drying apparatus. With this drying a uniform thin film having a uniform thickness of about 198 µm and a dry coating weight of 16.3 mg/cm² was produced.

This Example shows that an active ingredient, in this case a Cannabinoid, can be incorporated in the composition of the present invention to obtain an orally disintegrating film with the desired properties. The active ingredient forms a stable microemulsion with an oily medium and is then uniformly distributed in this microemulsion within the polymeric matrix.

**Table 3. Compositions of the orally disintegrating thin film containing CBD as active ingredient.**

| Example 4 | |
|---|---|
| Component | wt% in dry mass |
| CBD | 10.0 |
| Castor Oil | 6.0 |
| Lipoid P LPC 80 | 7.5 |
| SPAN 80 | 6.0 |
| Lycoat | 38.9 |
| Pullulan | 19.5 |
| Glycerol | 10.1 |
| Citric acid | 2.0 |
| Total | 100 |

### Example 5: Compositions and method of making an ODF with CBD dispersed in a Microemulsion in safflower oil

In Example 5 Cannabidiol (CBD) is incorporated as active pharmaceutical ingredient dispersed in a microemulsion within the polymer matrix of the orally disintegrating film. In Example 5 containing CBD as active ingredient, but a different oil and plasticizers is used compared to Example 4. This Example shows that a wide variation of components (in this case oil/plasticizer and emulsifier) can be used for the formulation of the present invention in combination with the above discussed combination of polymers and therefore this Example shows the scope of the present invention. The composition of Example 5 is summarized in Table 4. The orally disintegrating film of Example 5 was prepared according to the alternative general procedure **(****Figure 2****)** described above and the detailed manufacturing process is given below.

In a reaction vessel equipped with an overhead stirrer, 35.0 g Cannabidiol (CBD) is solved in 22.4 g propylene glycol and 42.0 g safflower oil at 45 °C. After that, 3.92 g Lipoid S 20 and 5.88 g Lipoid S 75 are added and the mixture is stirred at 45 °C and 700 rpm for 10 min. Subsequently, the 104 g Pullulan, 67.2 g Lycoat RS 720 and 420 g deionized water were added and the mixture was stirred with 700 rpm at 45 °C for 20 min.

After that, water was added to achieve the desired solid content (ca. 60 wt%) of the final coating mass. The resulting dispersion was degassed by stirring at 90 rpm at 45 °C for 2 h to remove air bubbles in the coating mass. Using a mechanical casting apparatus equipped with doctor blade assembly and convection heating chamber, the stable dispersion is cast onto a PET substrate at the rate of 0.075 m/min and a gap width of 500 µm, followed by drying at 70-90 °C using an IR-drying apparatus.

This Example shows that CBD can be incorporated in the polymeric matrix with a modified composition of the formulation. In this case the oily medium and the plasticizer was changed within the formulation of the present invention by keeping the combination of polymers in the composition, but still orally disintegrating films with the desired properties were obtained. In orally disintegrating film composition of Example 5 the active ingredient is uniformly distributed in a microemulsion within the polymeric matrix increasing in combination with the polymeric matrix the homogeneity and stability of the orally disintegrating films.

**Table 4. Composition of the orally disintegrating thin film of Example 5.**

| Example 5 | |
|---|---|
| Component | wt% in dry mass |
| CBD | 12.5 |
| Safflower Oil | 15.0 |
| Lipoid S 20 | 1.4 |
| Lipoid S 75 | 2.1 |
| Lycoat | 24.0 |
| Pullulan | 37.0 |
| Propylene Glycol | 8.0 |
| Total | 100 |

### Investigation of the properties of the different ODFs (Examples 1-5)

### • Physical properties

Although all examples were produced according to a similar manufacturing procedure for the preparation of the coating mass and for the coating process, the modification of the ratios of the two-hydrophilic polymers in the studied ODF examples provokes a strong variation of the material properties of the ODFs. For example, the dry coating weight (DCW) and the dry coating thickness (DCT) of the investigated ODFs are strongly increased for higher molar ratios of Lycoat concomitant with an increase of the densities of the orally disintegrating films (Table 5). Thus, Example 1 exhibits a much higher DCW (30.5 mg/cm²) and DCT (271 µm) compared to Example 2 (16.3 mg/cm², 149 µm) and Example 3 (14.7 mg/cm², 134 µm). These differences in the physical properties are in good accordance with the measured viscosities of the different coating masses, which are mainly influenced by the variation of different polymers, used for the casting process. For a coating mass with lower viscosity a thin film with higher thickness (associated with a higher DCW) is created by applying a constant gap width of the doctor blade during the process, while for a coating mass with high viscosity the thickness of the produced film is lower. In accordance to that, the viscosity of the coating mass of Example 1 is lower than that of Example 2 and that of Example 3. The above described behavior illustrates that the physical properties of the ODFs can be adjusted by variation of the ratios of the different polymers.

In the formulation of Example 4 CBD is included as active ingredient in the ODFs in contrast to the Examples 1-3 without active ingredient. Nevertheless, the properties of the ODFs containing the active ingredient are also influenced by the ratio of the used polymers. Furthermore, Example 4 demonstrates that an active ingredient, in this case a Cannabinoid, can easily be incorporated in the polymeric matrix of the orally disintegrating film by keeping the favorable properties of the thin-film.

**Table 5. Physical properties of the ODFs with different ratios of starch:pullulan.**

| | Ratio starch:pullulan | Dry coating weight [mg/cm²] | Dry coating thickness [µm] | Density [g/cm³] |
|---|---|---|---|---|
| Example 1 | 80:20 | 30.5 | 271 | 1.13 |
| Example 2 | 60:40 | 16.3 | 149 | 1.10 |
| Example 3 | 40:60 | 14.7 | 134 | 1.09 |
| Example 4* | 67:33 | 16.3 | 198 | 0.82 |

| | | | | |
|---|---|---|---|---|
| [*] Example 4 contains Cannabidiol (CBD) as active ingredient in contrast to the Examples 1-3. | | | | |

### • Disintegration

The disintegration of the thin films was *in vitro* characterized in a solution of phosphate buffered saline (pH = 6.8) at 25 °C to mimic the oral cavity. For the preparation of the dissolution medium 8 g of sodium chloride, 0.2 g of potassium dihydrogen orthophosphate and 2.66 g of disodium hydrogen phosphate dihydrate are dissolved into 1000 mL of demin. water and the pH is adjusted to 6.8 by addition of ortho-phosphoric acid (85%). 25 mL of buffer are filled into a petri dish and are placed onto an orbital shaker. The oral thin film is placed into the petri dish by using a tweezer and the samples are swiveled at a specific rate (75 rpm) to stimulate movement in the solution. The time for complete disintegration is recorded for 3 replicates of each oral thin film example. The reported disintegration times are the averages of the replicate measurements and are summarized in the following Table 6.

**Table 6. Disintegration times of the ODFs with different ratios of starch:pullulan.**

| | Ratio starch:pullulan | Disintegration time [min] |
|---|---|---|
| Example 1 | 80:20 | 12.8 |
| Example 2 | 60:40 | 2.8 |
| Example 3 | 40:60 | 1.5 |
| Example 4* | 67:33 | 2.3 |

| | | |
|---|---|---|
| [*] Example 4 contains Cannabidiol (CBD) as active ingredient in contrast to the Examples 1-3. | | |

These results indicate that changing the ratio of the hydrophilic polymers within the ODFs drastically influence the mechanical properties of the ODFs. An increase of the starch:pullulan ratio (Example 1), leads to much slower disintegration (12.8 min) compared to an ODF with a smaller amount of starch (Example 2: 2.8 min), while an decrease of the ratio (Example 3) leads to faster disintegration times (1.5 min). The use of a higher amount of starch leads to a higher stability of the ODFs concomitant with an increase of the disintegration time, because starch is a higher interconnected polymer compared to the more linear pullulan. This behavior illustrates that by changing the ratio of the molecular components of the formulation the properties of the orally disintegrating film composition can be adjusted.

Although in Example 4 an active ingredient is included in the formulation, the disintegration time is in the same range as for two examples without active ingredient (Example 2-3). This results further corroborate that the disintegration properties are mainly determined by polymers.

### • Rheological properties

The rheological properties were determined according to the DIN EN ISO 527-1 guideline (*Plastics - Determination of tensile properties*) and the USP general chapter <881>. For the determination of the ultimate tensile strength and the elongation at break of the different thin film formulations, bone-shape samples with a length of 115 mm and measurement cross-section of 6 ± 0.4 mm were cut out of the laminates of the ODFs. The samples were investigated with the tension meter T300 with a velocity of 5 mm/min at 23 °C and 50% RH. From these experiment the ultimate tensile strength and the elongation at break can be determined. The ultimate tensile strength, which is the maximum force applied until the breakage of the ODFs, is calculated by dividing the maximal force (N) the material can withstand through the cross-sectional area (mm²). The elongation at break (%) is the elongation at the time point when the sample starts to break. The values of the elongation at break and the ultimate tensile strength are summarized in Table 7.

The variation of the ratio of the polymers has a huge impact on the elasticity and stiffness of the material of the ODFs. For a higher pullulan content more ductile and elastic films can be produced. Accordingly, the elasticity determined for Example 3 (elongation at break: 109.1%) is much higher than that of Example 2 (107.9%) and Example 3 (103.3%).

This trend is also reflected in the in the tendency of the ultimate tensile strength, which is the maximum stress that the material can withstand while being stretched before breaking, of the investigated films. The studies of the tensile strength illustrate that the films with a higher pullulan content are more robust to applied stress than those with lower pullulan content. Consequently, Example 3 has the highest tensile strength (1.55 N/mm²) compared to Example 2 (1.03 N/mm²) and Example 3 (0.59 N/mm²).

These results illustrate, that by including a higher ratio of the more flexible polymer (pullulan), ODFs with a higher flexibility and lower stiffness are produced compared to the examples with a higher ratio of starch. Thus, the rheological properties of the orally disintegrating films are also determined by the physical properties of the used polymer.

**Table 7. ODFs with different ratios of starch:pullulan.**

| | Ratio starch:pullulan | Elongation at break [%] | Ultimate Tensile strength [N/mm²] |
|---|---|---|---|
| Example 1 | 80:20 | 103.3 | 0.59 |
| Example 2 | 60:40 | 107.9 | 1.03 |
| Example 3 | 40:60 | 109.1 | 1.55 |

### • Summary of the Examples

In conclusion, the studies of the above described examples, which are manufactured according to the procedure described in the present invention, demonstrate that the properties of the ODFs can be tuned by slight modifications, e.g. variation of the ratio of polymers in the formulation. In the described examples two different hydrophilic polymers were combined to form one thin film and to obtain novel properties, which are not accessible with previous methods. With the procedure described in the invention the production of ODFs with adapted and desired properties and features can be achieved.

Example 4 demonstrates that an active ingredient, in this example a Cannabinoid, can easily be incorporated within a microemulsion embodied in polymeric matrix of the orally disintegrating films of the present invention and still the properties of the ODFs can be controlled by the utilization of a combination of different polymers to achieve the desired medicinal product.

Finally, Example 5 shows that individual components (in this example the oily medium, plasticizer and the emulsifiers) can varied within the formulation by keeping the beneficial combination of the polymers (e.g. Lycoat and Pullulan) and still orally disintegrating films can be manufactured according to the procedure described in the present invention. This example demonstrates that the formulation tolerates a huge variation of components within the composition and illustrates the wide applicability of the present invention.

## Claims

1. An orally disintegrating film composition comprising an active ingredient and a polymeric matrix, comprising a combination of at least two water-soluble film forming polymers to form a polymeric matrix and wherein the active ingredient is uniformly dispersed within a microemulsion comprising an oil medium, water and at least one surfactant and optionally at least one cosurfactant, wherein the at least two water-soluble film forming polymers are a pregelatinized hydroxypropyl pea starch and pullulan and wherein the ratio of pregelatinized hydroxypropyl pea starch and pullulan is between 4:1 to 2:3.

2. The orally disintegrating film composition of claim 1, wherein the surfactant and/or cosurfactant is selected from the group consisting of polyglycolized glycerides, polyoxyethylene glycerides, polyethylene glycol-fatty acid esters, polyethylene glycol glycerol fatty acid esters, transesterification products of oils and alcohols, polyglycerized fatty acids, glycerol fatty acid esters, polyglycerol fatty acid esters, propylene glycol fatty acid esters, mono and diglycerides, polyoxyethylene-polyoxypropylene block copolymers, polyethylene glycol sorbitan fatty acid esters, sorbitan fatty acid esters, polysorbates, poloxamers, phospholipids, lecithin, phosphatidylcholines, phosphatidylethanolamines, phosphatidylinositols, phosphatidylserines, phosphatidic acids, and mixtures thereof.

3. The orally disintegrating film composition of claim 1 or 2, wherein the oily medium is selected from the group consisting of borage oil, coconut oil, cottonseed oil, soybean oil, safflower oil, sunflower oil, castor oil, corn oil, olive oil, palm oil, peanut oil, peppermint oil, poppy seed oil, canola oil, hydrogenated soybean oil, hydrogenated vegetable oils, glyceryl esters of saturated fatty acids, glyceryl behenate, glyceryl distearate, glyceryl isostearate, glyceryl laurate, glyceryl monooleate, glyceryl, monolinoleate, glyceryl palmitate, glyceryl palmitostearate, glyceryl ricinoleate, glyceryl stearate, polyglyceryl-10-oleate, polyglyceryl-3-oleate, polyglyceryl-4-oleate, polyglyceryl-10-tetralinoleate, behenic acid, caprylyic/capric glycerides, lauric acid, linoleic acid, linolenic acid, myristic acid, palmitic acid, palmitoleic acid, palmitostearic acid, ricinoleic acid, stearic acid, soy fatty acids, oleic acid, a-tocopherol, y-tocopherol, vitamin E, and vitamin A, and mixtures thereof.

4. The orally disintegrating film composition of any one of the preceding claims, wherein the active component is selected from the group consisting of pharmaceutical agents, cosmetic agents, bioactive agents and combinations thereof and wherein the active component is present in amounts of up to about 0.01% to about 50% by weight of the total composition, preferably 1% to about 30%, more preferably 10% to about 20%.

5. The orally disintegrating film composition of any one of the preceding claims, wherein the active ingredient is selected from the group comprising cannabinoids, tetrahydrocannabinol (THC), tetrahydrocannabinolic acid (THCA), cannabidiol (CBD), cannabigerol (CBG) and cannabinol (CBN), or mixture or combinations thereof.

6. The orally disintegrating film composition of any one of claims 1 to 5, wherein the film composition has a disintegration time of 300 s or less, preferably of 180 s or less, especially of 120 s or less, wherein disintegration time is measured according to the method described in Ph. Eur. Chapter 2.9.3.

7. The orally disintegrating film composition of any one of claims 1 to 6, wherein the film composition has an ultimate tensile strength of 1.8 N/mm² or below, preferably of 0.8 N/mm² or below, especially of 0.3 N/mm² or below, wherein ultimate tensile strength is measured according to the method described in USP general chapter 881.

8. A method for producing an orally disintegrating film composition according to any one of claims 1 to 7, wherein a preparation comprising the active ingredient dispersed in a microemulsion is mixed with a preparation of the first film-forming polymer, the pregelatinized hydroxypropyl pea starch, and a preparation of the second film-forming polymer, pullulan, by stirring with at least 200 rpm for at least 10 min at a temperature of at least 40°C.

9. A method according to claim 8, wherein stirring is performed with at least 200 rpm for at least 30 min at a temperature of 40 °C to 65°C, preferably of 45°C to 55°C.

10. A method according to claim 8 or 9, wherein the mixture is casted and dried at a temperature of at least 60°C, preferably at a temperature of 65°C to 100°C.

11. A method according to any one of claims 8 to 10, wherein the mixture is casted and dried to a film with a thickness of 10 to 800 µm, preferably of 200 to 500 µm, especially of 300 to 500 µm.

## Patentansprüche

1. Eine im Mund zerfallende Filmzusammensetzung, umfassend einen Wirkstoff und eine polymere Matrix, umfassend eine Kombination von mindestens zwei wasserlöslichen filmbildenden Polymeren, um eine polymere Matrix zu bilden, und wobei der Wirkstoff gleichmäßig in einer Mikroemulsion dispergiert ist, umfassend ein Ölmedium, Wasser und mindestens ein Tensid und gegebenenfalls mindestens ein Co-Tensid, wobei die mindestens zwei wasserlöslichen filmbildenden Polymere eine vorgelatinierte Hydroxypropyl-Erbsenquellstärke und Pullulan sind und wobei das Verhältnis von vorgelatinierter Hydroxypropyl-Erbsenstärke und Pullulan zwischen 4:1 bis 2:3 beträgt.

2. Die im Mund zerfallende Filmzusammensetzung nach Anspruch 1, wobei das Tensid und/oder Co-Tensid ausgewählt ist aus der Gruppe bestehend aus polyglykolisierten Glyceriden, Polyoxyethylenglyceriden, Polyethylenglykol-Fettsäureestern, Polyethylenglykol-Glycerinfettsäureestern, Umesterungsprodukten von Ölen und Alkoholen, polyglyzerisierten Fettsäuren, Glycerinfettsäureestern, Polyglycerinfettsäureester, Propylenglykolfettsäureester, Mono- und Diglyceride, Polyoxyethylen-Polyoxypropylen-Blockcopolymere, Polyethylenglykolsorbitanfettsäureester, Sorbitanfettsäureester, Polysorbate, Poloxamere, Phospholipide, Lecithin, Phosphatidylcholine, Phosphatidylethanolamine, Phosphatidylinositole, Phosphatidylserine, Phosphatidsäuren und Mischungen davon.

3. Die im Mund zerfallende Filmzusammensetzung nach Anspruch 1 oder 2, wobei das Ölmedium ausgewählt ist aus der Gruppe bestehend aus Borretschöl, Kokosnussöl, Baumwollsamenöl, Sojabohnenöl, Safloröl, Sonnenblumenöl, Rizinusöl, Maisöl, Olivenöl, Palmöl, Erdnussöl, Pfefferminzöl, Mohnöl, Canolaöl, hydriertes Sojabohnenöl, hydrierte Pflanzenöle, Glycerinester gesättigter Fettsäuren, Glycerylbehenat, Glyceryldistearat, Glycerylisostearat, Glyceryllaurat, Glycerylmonooleat, Glycerylmonooleat, Glycerylmonolinoleat, Glycerylpalmitat, Glycerylpalmitostearat, Glycerylricinoleat, Glycerylstearat, Polyglyceryl-10-oleat, Polyglyceryl-3-oleat, Polyglyceryl-4-oleat, Polyglyceryl-10-tetralinoleat, Behensäure, Caprylyin-/Caprinsäureglyceride, Laurinsäure, Linolsäure, Linolensäure, Myristinsäure, Palmitinsäure, Palmitoleinsäure, Palmitostearinsäure, Ricinolsäure, Stearinsäure, Sojafettsäuren, Ölsäure, α-Tocopherol, y-Tocopherol, Vitamin E und Vitamin A sowie Mischungen davon.

4. Die im Mund zerfallende Filmzusammensetzung nach einem der vorhergehenden Ansprüche, wobei der aktive Bestandteil aus der Gruppe ausgewählt ist, die aus pharmazeutischen Wirkstoffen, kosmetischen Wirkstoffen, bioaktiven Wirkstoffen und Kombinationen davon besteht, und wobei der aktive Bestandteil in Mengen von bis zu etwa 0,01 % bis etwa 50 Gew.-% der Gesamtzusammensetzung, bevorzugt 1 % bis etwa 30 %, noch bevorzugter 10 % bis etwa 20 %, vorhanden ist.

5. Die im Mund zerfallende Filmzusammensetzung nach einem der vorhergehenden Ansprüche, wobei der Wirkstoff aus der Gruppe ausgewählt ist, umfassend Cannabinoide, Tetrahydrocannabinol (THC), Tetrahydrocannabinolsäure (THCA), Cannabidiol (CBD), Cannabigerol (CBG) und Cannabinol (CBN) oder eine Mischung oder Kombinationen davon.

6. Die im Mund zerfallende Filmzusammensetzung nach einem der Ansprüche 1 bis 5, wobei die Filmzusammensetzung eine Zerfallszeit von 300 s oder weniger, bevorzugt von 180 s oder weniger, insbesondere von 120 s oder weniger aufweist, wobei die Zerfallszeit nach dem in Ph. Eur. Kapitel 2.9.3. beschriebenen Verfahren gemessen wird.

7. Die im Mund zerfallende Filmzusammensetzung nach einem der Ansprüche 1 bis 6, wobei die Filmzusammensetzung eine Zugfestigkeit von 1,8 N/mm² oder weniger, insbesondere von 0,8 N/mm² oder weniger, insbesondere von 0,3 N/mm² oder weniger aufweist, wobei die Zugfestigkeit nach dem in USP General Chapter 881 beschriebenen Verfahren gemessen wird.

8. Verfahren zur Herstellung einer im Mund zerfallenden Filmzusammensetzung nach einem der Ansprüche 1 bis 7, wobei eine Zubereitung, die den in einer Mikroemulsion dispergierten Wirkstoff umfasst, mit einer Zubereitung des ersten filmbildenden Polymers, der vorgelatinierten Hydroxypropyl-Erbsenstärke, und einer Zubereitung des zweiten filmbildenden Polymers, Pullulan, durch Rühren mit mindestens 200 U/min für mindestens 10 min bei einer Temperatur von mindestens 40°C gemischt wird.

9. Verfahren nach Anspruch 8, wobei das Rühren mit mindestens 200 U/min für mindestens 30 min bei einer Temperatur von 40°C bis 65°C, bevorzugt von 45°C bis 55°C durchgeführt wird.

10. Verfahren nach Anspruch 8 oder 9, wobei die Mischung bei einer Temperatur von mindestens 60°C, bevorzugt bei einer Temperatur von 65°C bis 100°C, gegossen und getrocknet wird.

11. Verfahren nach einem der Ansprüche 8 bis 10, wobei die Mischung zu einem Film mit einer Dicke von 10 bis 800 µm, bevorzugt von 200 bis 500 µm, insbesondere von 300 bis 500 µm gegossen und getrocknet wird.

## Revendications

1. Une composition de film orodispersible comprenant un ingrédient actif et une matrice polymère, comprenant une combinaison d'au moins deux polymères filmogènes hydrosolubles pour former une matrice polymère et dans laquelle l'ingrédient actif est uniformément dispersé dans une microémulsion comprenant un milieu huileux, de l'eau et au moins un tensioactif et facultativement au moins un cotensioactif, dans laquelle les au moins deux polymères filmogènes hydrosolubles sont un amidon de pois hydroxypropylé prégélatinisé et du pullulane et dans laquelle le rapport d'amidon de pois hydroxypropylé prégélatinisé et de pullulane est compris entre 4:1 et 2:3.

2. La composition de film orodispersible selon la revendication 1, dans laquelle le tensioactif et/ou le cotensioactif est choisi dans le groupe constitué de glycérides polyglycolysés, glycérides polyoxyéthylénés, esters d'acides gras de polyéthylène glycol, esters d'acides gras de glycérol de polyéthylène glycol, produits de transestérification d'huiles et d'alcools, acides gras polyglycérisés, esters d'acides gras de glycérol, esters d'acides gras de polyglycérol, esters d'acides gras de propylène glycol, mono et di-glycérides, copolymères séquencés polyoxyéthylène-polyoxypropylène, esters d'acides gras de sorbitan de polyéthylène glycol, esters d'acides gras de sorbitan, polysorbates, poloxamères, phospholipides, lécithine, phosphatidylcholines, phosphatidyléthanolamines, phosphatidylinositols, phosphatidylsérines, acides phosphatidiques, et leurs mélanges.

3. La composition de film orodispersible selon la revendication 1 ou 2, dans laquelle le milieu huileux est choisi dans le groupe constitué d'huile de bourrache, huile de coco, huile de coton, huile de soja, huile de carthame, huile de tournesol, huile de ricin, huile de maïs, huile d'olive, huile de palme, huile d'arachide, huile de menthe poivrée, huile de pavot, huile de canola, huile de soja hydrogénée, huiles végétales hydrogénées, esters de glycéryle d'acides gras saturés, béhénate de glycéryle, distéarate de glycéryle, isostéarate de glycéryle, laurate de glycéryle, monoléate de glycéryle, monolinoléate de glycéryle, palmitate de glycéryle, palmitostéarate de glycéryle, ricinoléate de glycéryle, stéarate de glycéryle, polyglycéryl-10-oléate, polyglycéryl-3-oléate, polyglycéryl-4-oléate, polyglycéryl-10-tétralinoléate, acide béhénique, glycérides capryliques/capriques, acide laurique, acide linoléique, acide linolénique, acide myristique, acide palmitique, acide palmitoléique, acide palmitostéarique, acide ricinoléique, acide stéarique, acides gras de soja, acide oléique, α-tocophérol, γ-tocophérol, vitamine E, et vitamine A, et leurs mélanges.

4. La composition de film orodispersible selon l'une quelconque des revendications précédentes, dans laquelle le composant actif est choisi dans le groupe constitué d'agents pharmaceutiques, d'agents cosmétiques, d'agents bioactifs et leurs combinaisons et dans laquelle le composant actif est présent en quantités allant d'environ 0,01% à environ 50% en poids de la composition totale, de préférence de 1% à environ 30%, plus préférablement de 10% à environ 20%.

5. La composition de film orodispersible selon l'une quelconque des revendications précédentes, dans laquelle l'ingrédient actif est choisi dans le groupe comprenant les cannabinoïdes, le tétrahydrocannabinol (THC), l'acide tétrahydrocannabinolique (THCA), le cannabidiol (CBD), le cannabigérol (CBG) et le cannabinol (CBN), ou leurs mélanges ou combinaisons.

6. La composition de film orodispersible selon l'une quelconque des revendications 1 à 5, dans laquelle la composition de film a un temps de désintégration de 300 s ou moins, de préférence de 180 s ou moins, en particulier de 120 s ou moins, le temps de désintégration étant mesuré selon la méthode décrite dans la Ph. Eur. Chapitre 2.9.3.

7. La composition de film orodispersible selon l'une quelconque des revendications 1 à 6, dans laquelle la composition de film a une résistance à la traction ultime de 1,8 N/mm² ou moins, de préférence de 0,8 N/mm² ou moins, en particulier de 0,3 N/mm² ou moins, la résistance à la traction ultime étant mesurée selon la méthode décrite dans le chapitre général 881 de l'USP.

8. Un procédé de production d'une composition de film orodispersible selon l'une quelconque des revendications 1 à 7, dans lequel une préparation comprenant l'ingrédient actif dispersé dans une microémulsion est mélangée avec une préparation du premier polymère filmogène, l'amidon de pois hydroxypropylé prégélatinisé, et une préparation du second polymère filmogène, le pullulane, par agitation à au moins 200 tr/min pendant au moins 10 min à une température d'au moins 40°C.

9. Un procédé selon la revendication 8, dans lequel l'agitation est effectuée à au moins 200 tr/min pendant au moins 30 min à une température de 40°C à 65°C, de préférence de 45°C à 55°C.

10. Un procédé selon la revendication 8 ou 9, dans lequel le mélange est coulé et séché à une température d'au moins 60°C, de préférence à une température de 65°C à 100°C.

11. Un procédé selon l'une quelconque des revendications 8 à 10, dans lequel le mélange est coulé et séché en un film d'une épaisseur de 10 à 800 µm, de préférence de 200 à 500 µm, en particulier de 300 à 500 µm.
